# EUROPEAN PATENT APPLICATION

(11) **EP 1 842 858 A2**
(43) Date of publication of application: **10.10.2007**
(21) Application number: 07112139.6
(22) Date of filing: 24.04.2002
(51) Int. Cl.: C07K 16/00

(54) **Methods and compositions for treating oral and esophageal lesions**

(30) Priority: 24.04.2001 US 286240 P
(62) Divisional of application: 02764324.6
(71) Applicant: THE GENERAL HOSPITAL CORPORATION, Boston, MA 02114 (US)
(72) Inventor: Podolsky, Daniel, K., Wellesley Hills, MA 02481 (US)
(74) Representative: Gervasi, Gemma

(57) **Abstract**

The invention features methods and compositions for treating or preventing lesions of the upper elimentary canal, particularly oral aphthous or mucositis lesions. Intestinal trefoil peptides are administered in effeective concentrations either alone or in combination with different therapeutic agents.

SEQUENCE LISTING

<110> The General Hospital Corporation

<120> Methods and Compositions for Treating
  Oral and Esophageal Lesions

<130> 50206/002WO2

<150> US 60/286,240
<151> 2001-04-24

<160> 9

<170> FastSEQ for Windows Version 4.0

<210> 1
<211> 73
<212> PRT
<213> Homo sapien

<400> 1
Met Leu Gly Leu Val Leu Ala Leu Leu Ser Ser Ser Ser Ala Glu Glu
 1               5                  10                  15
Tyr Val Gly Leu Ser Ala Asn Gln Cys Ala Val Pro Ala Lys Asp Arg
            20                  25                  30
Val Asp Cys Gly Tyr Pro His Val Thr Pro Lys Glu Cys Asn Asn Arg
        35                  40                  45
Gly Cys Cys Phe Asp Ser Arg Ile Pro Gly Val Pro Trp Cys Phe Lys
    50                  55                  60
Pro Leu Gln Glu Ala Glu Cys Thr Phe
65                  70

<210> 2
<211> 84
<212> PRT
<213> Homo sapien

<400> 2
Met Ala Thr Met Glu Asn Lys Val Ile Cys Ala Leu Val Leu Val Ser
 1               5                  10                  15
Met Leu Ala Leu Gly Thr Leu Ala Glu Ala Gln Thr Glu Thr Cys Thr
            20                  25                  30
Val Ala Pro Arg Glu Arg Gln Asn Cys Gly Phe Pro Gly Val Thr Pro
        35                  40                  45
Ser Gln Cys Ala Asn Lys Gly Cys Cys Phe Asp Asp Thr Val Arg Gly
    50                  55                  60
Val Pro Trp Cys Phe Tyr Pro Asn Thr Ile Asp Val Pro Pro Glu Glu
65                  70                  75                  80
Glu Cys Glu Phe

<210> 3
<211> 106

<212> PRT
<213> Homo sapien

<400> 3

<210> 4
<211> 222
<212> DNA
<213> Homo sapien

<400> 4
atgctggggc tggtcctggc cttgctgtcc tccagctctg ctgaggagta cgtgggcctg 60
tctgcaaacc agtgtgccgt gccagccaag gacagggtgg actgcggcta cccccatgtc 120
acccccaagg agtgcaacaa ccggggctgc tgctttgact ccaggatccc tggagtgcct 180
tggtgtttca agcccctgca ggaagcagaa tgcaccttct ga                   222

<210> 5
<211> 255
<212> DNA
<213> Homo sapien

<400> 5
atggccacca tggagaacaa ggtgatctgc gccctggtcc tggtgtccat gctggccctc 60
ggcaccctgg ccgaggccca gacagagacg tgtacagtgg ccccccgtga aagacagaat 120
tgtggttttc ctggtgtcac gccctcccag tgtgcaaata agggctgctg tttcgacgac 180
accgttcgtg gggtcccctg gtgcttctat cctaatacca tcgacgtccc tccagaagag 240
gagtgtgaat tttag                                                  255

<210> 6
<211> 390
<212> DNA
<213> Homo sapien

<400> 6
atgggacggc gagacgccca gctcctggca gcgctcctcg tcctggggct atgtgccctg 60
gcggggagtg agaaaccctc cccctgccag tgctccaggc tgagccccca taacaggacg 120
aactgcggct tccctggaat caccagtgac cagtgttttg acaatggatg ctgtttcgac 180
tccagtgtca ctggggtccc ctggtgtttc caccccctcc caaagcaaga gtcggatcag 240
tgcgtcatgg aggtctcaga ccgaagaaac tgtggctacc cgggcatcag ccccgaggaa 300
tgcgcctctc ggaagtgctg cttctccaac ttcatctttg aagtgccctg gtgcttcttc 360
ccgaagtctg tggaagactg ccattactaa                                  390

<210> 7

<211> 3280
<212> DNA
<213> Homo sapien

<400> 7
atgctggggc tggtcctggc cttgctgtcc tccagctctg ctgaggagta cgtgggcctg 60
tgtgagtact gccctgactg ccccggtggc agggtgggcg tgaagggaag ggatccagga 120
taagggggga ttctgcattc atttaataat ggccacctgt cacatataca ctttttcctg 180
cgctagccct ttgaagtggg tctttattgt ccccatttca cagacaagga aaccgaggct 240
cagagaaagt taacaactta tccaaggcag ccctgcccag tctgtgttga aatcagggtt 300
tgagcctgag cccatcccct atgaccccat agccatcttt gctggagatt tctaaattac 360
aatataggtc tttatgcatt gttccacatt tacaaagaaa aaggaaagat gcaggagaaa 420
aaccctgact tcagaacact gtcaataccg gcaggcacaa ggttcattta gccattgcat 480
agcaaccctg ccatggggtg tggctgctcc attaacccaa gtttgaagga atgagggcat 540
ggcttttatc tgggtgtctt ctgagcaggg tcaaaggcag tggttcccga acttgcagcc 600
cattagaatc acctggagag ctttaaaaat cctaatgctt ggggcacacc agttacatca 660
gggcatctcc aggcaagatc caggcctcag ctgttttgtt ttgagatagc cttgctttgt 720
cactcactgc tggagtgcag tggcacaatc tcagctcact gcaacctccg cctcctgggt 780
tcaagcaatt cttgtgcctc ggcttcaagt agctgggatt acaggcatgc accaccatgc 840
ccagctaatt ttttggattt ttagtagaga tggagtttcg ctatgttggc caagctggtc 900
tcaaactcct ggcctcaagt gatcctcctg ccttggcctc ccaaagtgct ggaattacag 960
gtgtaagcca ccatgcccag ccaacgtcag tcatttttaa agctctgcag ctgattccag 1020
tgtgagcgaa gtttggatgc caggaggata agcaattacg gactgggagc aagagaaggg 1080
aatgtaagac actgcacgtg attgccattt tcctaaggaa atactcagtt cgttaatgaa 1140
acgcagtgaa cttctgctgc acatacagac atagaggctt gcctgaaaca tgaaaatatt 1200
ggggactgaa ggatgtcccg ggagggtggg acatgctcaa caattcagga aggggagatg 1260
cagaaaaaag tgaaaagcag gcagcatgcg ttgcaatgat ctctatggcg tgtgcctctc 1320
ctgtcacggt tttcatttaa aacaaagggg caaggttttg ttggtcaaac aatgaagggt 1380
aactttgttt ctgggttcaa gggaccccag attccccagg ggttcctgcc agctggaagg 1440
tacccaggtc cgtatgtgac ttcccgagaa ggtgataaga gcgtgccaag gagaaagaca 1500
cttaggcaaa tggccagagt ccccgagctg agcatttaac agactgcctc tctttaaata 1560
ttcacaggga aagtgcatct tcctaagggc gagggtttca gcagtggttg aactcggcgg 1620
ggtggggcgg agcgggagga tgcaaacttg caaagtgaag caaacacact caccgcagcc 1680
cagcaagggc tctggcagct gacagggctt tgtctgggac agctgcaaac cagtgtgccg 1740
tgccagccaa ggacagggtg gactgcggct acccccatgt cacccccaag gagtgcaaca 1800
accggggctg ctgctttgac tccaggatcc ctggagtgcc ttggtgtttc aagcccctgc 1860
aggaagcagg taaggcccca gtggcatcgt ggtctgggcc cagccccata aggcaggggg 1920
tctcagggcc tccctgtcct ttctgggctg gagatggagg cacaaggacc ccaggaagcc 1980
acacacacac acctgttcca aggcctcaga gcagaggctt cacacttagg gcagccatgg 2040
ccaggggctg tcctcttctg tcccctttat gtaaaacata aaagcaattg tttcaaaaag 2100
gtgttcaaaa tgatggcatc gcatagaggg aactgattta gtaactattc ttgagagaag 2160
tggaaacgca taggtgtgga aagccgggcc gacttttggg ctgtttttgc aaatcggccc 2220
cccagagtct tgtcatttgt ggcatcccct acacagacgg caggcggtcc cagccctaga 2280
cgtcaggcct cggtgccaca ccccacctcc cccactctgc cccccacaag ggtcatctcc 2340
tctccctctc tctgccgtgg tggagggcag gtgcagggca accaccctgg gggttccctc 2400
cccaggggcg gagagcctgc gtgctgtgcg ggtaacagat ggccctgcac acgggtttgc 2460
caccctggct ccaccaggct tagctgcccc acatcgtggg tggggcgatt ggctataagc 2520
catctgccat gtccaagtgc cagctcagcc cccacgaagg ccgcacctgc gtgaggtacc 2580
ttcctggaac cagcatccag aggggcctct cttgcccttt gtcctagggt gaaatgcggg 2640
aggctgagtc ctgctggccc cggctccctg atcaatgatg ggcccctgcc cagggcctcc 2700
cttcaccctc cccagcaagt ccagggtagg ggtgggggtg ggggtccaga gaaggccagg 2760
agagagaggg gtctggctac tgtccactgc cggtcctgtt ccttcagctc cactggaact 2820
acactctcct ctgagtgcca gccatggccc tgccaaggcc catctcgctt gttatctgcc 2880
tgatccctgg gtcccactat cttgcttagc aacccgaggt gggaatcttg gctattcccc 2940
catgtggtgg ggactcaaca ctccccggtg actctgggga ggaggcagca ctaggtgctg 3000
gccttggagc ctgccctgac cttgggaagc tgggcagcgt gggtggagag agactgctca 3060
cacaagcctt tgctctgttt gcagaatgca ccttctgagg cacctccagc tgcccccggc 3120
cgggggatgc gaggctcgga gcacccttgc ccggctgtga ttgctgccag gcactgttca 3180

tctcagcttt tctgtccctt tgctcccggc aagcgcttct gctgaaagtt catatctgga 3240
gcctgatgtc ttaacgaata aaggtcccat gctccacccg 3280

<210> 8
<211> 4623
<212> DNA
<213> Homo sapien

<400> 8
dcccctgggt gcagctgagc tagacatggg acggcgagac gcccagctcc tggcagcgct 60
cctcgtcctg gggctatgtg ccctggcggg gagtgagaaa ccctgtaagt gaaggagagg 120
gtctttttat gtgctttctt tatttctctt aaagaaaaaa aaaaagcaca accataaatt 180
aacttgagag ggggaatggc tataaaggca tctggcaatg tgtgttgttc acatggattt 240
tgccactgct caggagggtg gctccaagaa gggcctccct cctagggaaa ggctgagtga 300
cggcaggtgt cagcgggccc cgtgtcgggc caggagggca ttcccaccaa gggtccttgg 360
agtcccagag cactcacctc tcgcctggat cttggccttg ggtccatctg ttcaccctcc 420
tctaggaggg ttttgttttt gtttttttcc gagacaggat ctggctttgc cgcccaggca 480
ggagtgcagt ggtgtgatct tggctcactg caacctctgc ctcccaggct caagtgatcc 540
tcccacctca gccgcctgag tagctgaaac cacagttgtg gaccatcatg cccggccaat 600
tttttttttt gtattgtttg tagagatggg gtttcgacat gttgcccagg atggtcttga 660
actcctgagc tcaagcaatc tgcccgcctc ggcttcctaa agtgctggga ttataggtat 720
gagccaccat gcctggcttt tttttttttt tccttttaaa ctaatataac aatttcagca 780
aagccctatc ggcttctcag gaggaaaccg cattgcttaa atatgggcaa gataagactt 840
tgtgtttctc tatgtggcaa caagacagta gaggcatccc ctagaacctc tgagagaagg 900
agcagtgtgg tctggggtac cagggtgggg ccgactgagg gtctttccac agccccctgc 960
cagtgctcca ggctgagccc ccataacagg acgaactgcg gcttccctgg aatcaccagt 1020
gaccagtgtt ttgacaatgg atgctgtttc gactccagtg tcactggggt cccctggtgt 1080
ttccaccccc tcccaaagca aggtaatctt ccagggaatc ttcctgggcc agcagctggc 1140
aacccaggac ccagcttcac aggcggagcc cagagcaggg gccggaggag gcccagttgc 1200
tagtctaggg ttagcctggg tgggttagtc tcgagctagc cccggttggt tagtctgggg 1260
ctagcccagg ttggttagtc tagagctagc ccaggttggt tagtctgggg ctagcccagg 1320
ttggttagtc tggggctagc ccaggttggt tagtctaggg ctagtgtagg ctagttagtc 1380
taaggctagc ccaggttggt tagtttggag ctagcgcagg ttggttagtc tggggctagt 1440
agcccaggtt ggttagcctg gagctagccc aggttggtta gtctagggct agcgtaggct 1500
ggttagtctg gggctagccc aggttggtta gtctggagct agcccaggtt ggttagtctg 1560
gggctagtag cccaggttgg ttagtctggg gctagcccag gttggttagt ctagggctag 1620
tgtaggctag ttagtctagg gctagcccag gttagttagt ttggagctag cacaggttga 1680
ttagtctggg gctagtagcc taggttggtt agtctggagc tagcccaagt tggttagtct 1740
agggctagca taggctggtt agtctggggc tagtagccta ggtttgttag tctggagcta 1800
gcccaggttg gttagtctag ggctagcgta ggctggttag tctagggcta gcccaggttg 1860
gttaatcgga gctagcccag gttggttagt ctggagctag cccaggttgg ttagtctgag 1920
gctagtagcc caggttggtt agtctggggc tagcccaggt ttgttagtct ggagctagcc 1980
caggttgttt agtctggagc tagcccaggt tggttagtct gggactagcc tggactgcta 2040
gtctagaggt agcctagagg actgctagtc tagaggtagt ctagggctag cccaggttgg 2100
ttagtctggg gctagcccat gttggttagt cttagactag cctggactgc tagtctagag 2160
gtagcccagg ttgtttagtc tggtactagc ctggactgtt agtctagagg tagcccaggt 2220
tggttaggtt ggttagtctg ggactagtct ggactgttag tctagaggta gcccaggttg 2280
gttagtctgg gactagcctg gactgttagt ctagaggtag cccagattgg ttagtctggg 2340
actagtctgg actgctagtc tagaggtagc ccaggttggt tagcctgggg ccagcctgga 2400
ctgttagtct agaggtaacc caggtcagcc aacagtgaga tgaaaatttc ccacctaccc 2460
tgtttctaca ctgttagttc tttcaacaga catgtgtgtg tggagccatc agttttactt 2520
tagttgagaa aaaaatatat atatatatag taggtctcct ctagtttttg aagtgtgact 2580
tctgaagaag cttccatggg gaaatgaagg tatttaatag gacagcagta acataagggc 2640
tgacagccct caaatgttag ggaaggaagt gaagccttct agggttcttt gggagtgagt 2700
tttatgttag tgcacgggat caggacccaa gttgtaacgc cgacgagtgc tcaaaggaag 2760
gttgtgtgtg tgtcgtgcac ctgtgtgcgt ggaaccaggc acgtcctctg gagaaggagg 2820
attcatcccc aagattgttg ctgggaggct tgctgggccc cgcagggaaa ccaggcagat 2880
ggtggattgt tcacgagcgc ccactgaatg gcagtgtctt tgggaatcaa taccatgtcc 2940

aaacgctttc catcttacca aggtgcccac aaaccttttc tcatcttggc ccgggggacc 3000
accccattta ctgagaacac tgagtcccga gaggcaaaat gatttcccca aggcggggga 3060
ctccagagct tctgactgtg accaccccac atgggcccca ccttcgcgga ggacaggcca 3120
gccaagcgtc gctggggccg acacttccac agtccccggg ggaggcggtc ccaggggccg 3180
acacttccac agtccccggg ggaggccgtc ccgggggatg ctgccccagg cagcacctca 3240
tgatccacgg aggctgcaaa tcagcgctgc tctcagagga ggaaggggtg gagctttcca 3300
gggcacagca ggcctgactg ggtctcggtg ctgtgcctgt cccatggcag agtcggatca 3360
gtgcgtcatg gaggtctcag accgaagaaa ctgtggctac ccgggcatca gccccgagga 3420
atgcgcctct cggaagtgct gcttctccaa cttcatcttt gaagtgccct ggtgcttctt 3480
cccgaagtct gtggaaggta acgtcgctgt gggactctct gtctggttcc cggacaccat 3540
gattcctcct ccgtccgtag aggtggggtg cagggagggg agctgcctcg cagcctcagt 3600
gccatcgagg ccagggcccc tgcctcctat gggattctga aggcaattcc agaatgttct 3660
tggcaaagac agcgtctttt caataagttt atagcctcca gcattgccac tgcgtcatct 3720
gtgatggctc tagaaacagc ggctcatccc tgttgcctcc ccaggtgttg caacgttcag 3780
aggcgttgcc tgttttattg caagcccatc tgcatttgga ggctactgag tgtcttgcac 3840
tgtgctgggt accagagagg gcccaactca agcagacctg gccccttctc ccgtggcttc 3900
cccgttctcc cccacatgac cccgaatgac aaacctcatc cacaacgtcc tgctccgggc 3960
agtcccggga gggtcccgcc ggcagaggtg aacgggtcca cttctcccac ccgcttagtg 4020
atagtgtgtt cctgactcgg agtgtggcga ggtaaaaaaa gaccaagcag atccaggaaa 4080
atggggaaag agctactggc ccttgaagga tgccttttct tttccttttg ttaggatatc 4140
aaagcactcc aaagagcgaa atatttcatg ttcaggattt tccgagtgat tttttttatg 4200
tgacctaaag gtccacctag aaaatgttca cttgtctggg gagaatgcgc cccacagagg 4260
aaactctggc ctggggtggg aagatttggt ccctttacac cccctccccg ggaaaggagc 4320
tccttcttca gtaggaagct cctgggcaaa gtgatgcacg cccaccccag cttcgcagcc 4380
taggcactcc catttctggg gttcccttac caaccatctt gcatttaaac ttctagactg 4440
ccattactaa gagaggctgg ttccagagga tgcatctggc tcaccgggtg ttccgaaacc 4500
aaagaagaaa cttcgcctta tcagcttcat acttcatgaa atcctgggtt ttcttaacca 4560
tcttttcctc attttcaatg gtttaacata taatttcttt aaataaaacc cttaaatctt 4620
gct 4623

<210> 9
<211> 4252
<212> DNA
<213> Homo sapien

<400> 9
atccctgact cggggtcgcc tttggagcag agaggaggca atggccacca tggagaacaa 60
ggtgatctgc gccctggtcc tggtgtccat gctggccctc ggcaccctgg ccgaggccca 120
gacaggtaag gcgtgcttct tcctgctctg tggggccaca gccagctctg gcagcctccg 180
ccaggagcca ctgttttaca tacatatttt tgagcacctg ttttgtgcca ggtgctgttc 240
taggccctta aaagtatatc caatttacag gatcggcaaa agcaggtgga gagtaactca 300
gggtggcagg gcccccggag accttcgaga agtgcgacga ggagggggct gccttcagtc 360
ggggctgttt tcctgtgtta ggaagactat acaatcctcc caagtgtcat gtttcaaaga 420
ggaagtgttg gcgtggggtc tcagaatagt gcttttgact gttcatgcca acatctcccc 480
caggggcaga ccctcccaag gcccatccag ataggcccaa atgccggtcc cagtgatggc 540
cacctgggag accctctccc acaggcccga atgcccgtcc cagtggtggc caactgggag 600
accctctcct acaggttcct gggctcccct gggatccatg ctctgggagt caaagccacc 660
tctctcatga gtgcgtggct ggcaacccat attccctggt gttgtcaagt ggatcggttg 720
ccctgggtcc ttctagggag tggaggagga ggccattctt gcttccttgg gaagtgtttg 780
catctcaact cctttacctg cagaatggat caacggtctg ccctagggct gtcaggaaat 840
gctgtgtggc agcatctgcg acttgcactt tgccagctgt ggggagctga ataacttatt 900
tgccgttatt aggtacagtt tcaaggtggg ggcaggagaa agggctttct acgtttccaa 960
agcaagggtt tccagagagg cctgaagagg gagcgcccag tggtgctgtc cgtgccccca 1020
ctgccctcca gccacctctt gatctctgct gtggggtacc gggcctgagg ggtgggcttg 1080
ggcagcgtag aagagcagcc agcattgggc tgcagtggga agacccccaa gcccatggca 1140
gggagcgggg gagctttgga acccgagaga ggaagtggcc tcggtgtaca gaacgaactg 1200
ggtgggtccc cgtgctggcc acccccaggc ccatctgcct gcgcccttgc ccccacccca 1260
gcccccagct ctgccccctg tgctgtggga tcacagaggc cgtggcaaac tcccctcccc 1320

accccacaca ccctctggct caaggctcag agcgtctttg cgggtcactc aggtccatga 1380
tcctgttaca actgaaatct agaaaattgt gattacagtt tagtgcattc gtgtgtggaa 1440
accatttcca tttatttcca tcatgcgaca aagacaaagc gggtgggcaa gacagagtct 1500
gccggaggca gagcaccggg gctggaaatc ttcctccctg aggaggaaac ccccccgacc 1560
cccaggatga tgatcctccc tcaccacggg gcctctcttg acccccacag tgtcccgggg 1620
gtgggcgatg atcaccttca cgtcgcgatg gatccagacc ccaggagggc aaggttccca 1680
tggaagctgc tgggcagcgg gagctgaaca cggatccttc ccagcaagcc aggaacactt 1740
tctccaaaga catctcgagg cagtccctga tagcaaagca gacaagagaa cagcccctct 1800
cggcctcccc tggggcgccc tcacctgagc cagtgtggcc agactgagtt cctcccctcc 1860
tatgccccaa ggcagggaca gggaccggag ggtgctctgg gctcctcttt caccccctgc 1920
tgcaggctgt caaccaccag atcctaatag gttgctttct gagacctttg attccgcgga 1980
gctcagagcc tgaagctctg gtgttagaac ctcttgcata agatcctgcg gcagccccca 2040
gccagcccca tctgtccacg tgtcttcctc ctctagatcc ctttcctcac tgccctgctt 2100
caagctgttt cacagcttgt accctctgtc ggctcctcct agaccacccc acccggtcct 2160
ctcaccttac ctgcaatggg tttccacctc ctgaacacac ctgggtctct ggaatggcct 2220
ttgcccatgc ggctccatct tcacctggtg aacctcctcc tgcagggagc ccccctgctt 2280
tgttcaacct gcttgtcatt ggcctctccg gggagtgccc tacccccgtg gttaccctgg 2340
gcaccctggg acgatggcct tgcgttgtct cgcacatgtt cttgcctttc tcctccatca 2400
gatccttaga ctcttttttt tttttttttg agatggagtc ttgctctgtc actcaggctg 2460
gagtgcaatg gtgcgatctt ggctcactac aacctctgcc tcctgggttc aagtgattct 2520
cctgcctcag cctcccaagt agctgggatt acagacgtgt gccacaatgc ccgcctaatt 2580
ttttgtattt ttagtagaga tggggcttca ccattttggt caggctggtc ttgaactcct 2640
gacctcaagt gattcacctc cttcagcctc ccaaagtgct gggattacag gcatgagcct 2700
gggcccagat atttagactc ttattaatga cttctctggt tttaatttct gggtctctct 2760
cacctggcac agtgcctggc ttttgccatg ctagctccca cttctcatgc acacaaatgg 2820
tgctcagtaa atatttatgt attgagtaaa atttaataat catttgttga aattaaaaag 2880
tgaataaata agttacctag aaagatgcaa agtccacaaa cctggggcac cttgcatttt 2940
ccctgagcgt aatgtttgca catcaggatg tgaggaccac gtctccctct catgtcctga 3000
gggttttata tccgcctcac tggacagttg ctgatgtcat tggagaagga agctggatgg 3060
gtgtgtgcat gataacatca aggaattcag cccacaactt actttgcttc ttacctgtgc 3120
actttcagag acgtgtacag tggccccccg tgaaagacag aattgtggtt ttcctggtgt 3180
cacgccctcc cagtgtgcaa ataagggctg ctgtttcgac gacaccgttc gtggggtccc 3240
ctggtgcttc tatcctaata ccatcgacgt ccctccagaa ggtatggcct ttttatacga 3300
tgggttctga agatttagaa ttagttagaa aagtcattta agactacaga ggctctgatc 3360
agcatcacca gctatgcctt tacacagagt cacggccgcc agtggtggtg caatggggta 3420
gcctgagtca ggctgcattc aggtccagga atagaaaggc agggctaagg gacttgggaa 3480
gaaacctgat ttccccccgg cttctcttca catctctaac caaaagcctg ggaagagcca 3540
ctgttggtaa cgctttctag cttgcctagg atagaggggg aaggcatgac gaaatctgaa 3600
gacatttcat gtattctttt tttttttttt tttttgaaat ggagtctcgc tccgttgccc 3660
ctgagctgga gtgcaatggt gcgatcttgg ctcactgcaa tctctgcctc ctgagttcaa 3720
cctcagcttc ctagtagctg agattacagg tgtgtgccac tacgcccagc taaatttttt 3780
ttgtattttt agtatagacg gggtttcacc atgttggcca gaccggtctt gaactcttga 3840
cctcaggtga tctgcccgcc tcagcctccc agagagctgg gattacaggc gtgagccacc 3900
gtgcccggct gacagttcat gttttctaaa gaatgtgcct atggatactt taaagtaaaa 3960
actctgtaat tgtttaaatg tgaaagaaaa tgtttatcct cactaaagca tctctttctc 4020
cctccccctc acccctgtag aggagtgtga attttagaca cttctgcagg gatctgcctg 4080
catcctgacg cggtgccgtc cccagcacgg tgattagtcc cagagctcgg ctgccacctc 4140
caccggacac ctcagacacg cttctgcagc tgtgcctcgg ctcacaacac agattgactg 4200
ctctgacttt gactactcaa aattggccta aaaattaaaa gagatcgata tt 4252

## Description

### Field of the Invention

This invention provides methods and compositions for treating lesions of the upper alimentary canal including the oral cavity and esophagus.

### Background of the Invention

Oral mucositis is the destruction of the oral mucosal epithelium which results in erythema, ulcerations, and pain in the oral cavity. Mucositis often arises as a complication of antineoplastic therapy such as cancer chemotherapy or radiotherapy. The painful ulcerative lesions of mucositis can cause patients to restrict their oral intake of food and liquids; as a result, they lose weight and suffer from dehydration. Severe mucositis can necessitate the de-escalation or the complete interruption of anti-neoplastic therapy. Chemotherapy or radiotherapy can also disrupt mucosal epithelium more distally in the gastrointestinal tract including the esophagus, stomach, and small and large intestines, resulting in pain and organ dysfunction (i. e., diarrhea).

The mucositis lesions are also sites of secondary infections, acting as portals of entry for endogenous oral microorganisms; a particularly serious concern in patients who are immunocompromised. Mucositis is therefore a significant risk factor forchronic debilitating local infections (e.g. yeast (Candida) infections) as well as life-threatening systemic infection (septicemia).

Patients with mucositis and neutropenia have a relative risk of septicemia that is at least four times greater than that of individuals without mucositis.

Aphthous ulcers of the mouth (or aphthous stomatitis) are a common and painful problem; approximately 10% of the population suffers from these mouth sores at one time or another. The cause of outbreaks of aphthous sores are not well understood, although they tend to be associated with stress and minor injury to the inside of the mouth. No satisfactory treatments are available, although topical application of steroids provides relief for some patients.

### Summary of the Invention

This invention features a method for treating a lesion of the upper alimentary canal in a mammal by administering to the mammal a therapeutically effect amount of an intestinal trefoil peptide. Preferably, the mammal is a human.

In preferred embodiments, the intestinal trefoil peptide is spasmolytic polypeptide (SP), pS2, or intestinal trefoil factor (ITF). More preferably, the intestinal trefoil peptide is ITF.

Lesions of the upper alimentary canal such as mucositis, aphthous stomatitis, and gingivitis can be treated by the methods of this invention.

Additionally, lesions of the upper alimentary canal that result from antineoplastic therapy (i. e., chemotherapy or radiotherapy),Behcet's Disease, biopsy, surgery, tumor resection, thermal or chemical burns, tooth extraction, trauma from any cause, or lesions caused by microbial (i. e., bacterial, viral, or fungal) infection are also amenable to treatment.

In preferred embodiments, the patient is also administered a second therapeutic agent. Preferred second therapeutic agents include anti-inflammatory agents, antibacterial agents (i. e., penicillins, cephalosporins, tetracyclines, or aminoglycosides), antifungal agents (i. e., nystatin or amphotericin B), antiviral agents (i. e., acyclovir), topical antiseptics (i. e., povidone-iodine), analgesics (i. e., lidocaine or benzocaine), or steroids (i. e., triamcinolone or hydrocortisone).

Preferably, the second therapeutic agent is administered within 3 days, 1 day, 12 hours,1 hour, or simultaneously with the intestinal trefoil peptide. The second therapeutic agent can be present in the same pharmaceutical composition as the intestinal trefoil peptide.

The invention also features pharmaceutical compositions suitable for delivering an intestinal trefoil peptide to the upper alimentary canal. Preferably, the pharmaceutical composition is an oral spray, an oral rinse (mouthwash), an ointment, a paste, a cream, a gel, chewing gum, a chewable tablet, a lozenge, or a bioerodable film. In one embodiment, the pharmaceutical compositions use bioerodable microspheres to encapsulate one or more of the therapeutic agents. In preferred embodiments of an oral spray, rinse, ointment, paste, gel, or bioerodable film, a mucoadhesive or viscosity-enhancing agent is present.

In other preferred embodiments, the intestinal trefoil peptide of the pharmaceutical composition is SP, pS2, or ITF. More preferably, the intestinal trefoil peptide is ITF. In other preferred embodiments, the pharmaceutical composition further contains a second therapeutic. Preferred second therapeutic agents include anti-inflammatory agents, antibacterial agents (i. e., penicillins, cephalosporins, tetracyclines, or aminoglycosides), antifungal agents (i. e., nystatin or amphotericin B), antiviral agents (i. e., acyclovir), topical antiseptics (i. e., povidone-iodine), analgesics (i. e., lidocaine or benzocaine), or steroids (i. e., triamcinolone or hydrocortisone).

By "intestinal trefoil peptide"is meant all mammalian homologs of human spasmolytic polypeptide (SP; also known as TFF2), human pS2 (also known asTFF1) and human intestinal trefoil factor (ITF ; also known as TFF3), and biologically active fragments thereof. Homologs of the trefoil peptides have, preferably, 70% amino acid identity to the human sequence, more preferably85% identity, most preferably 95%, or even 99% sequence identity. The length of comparison sequences will generally be at least about 10 amino acid residues, usually at least 20 amino acid residues, more usually at least 30 amino acid residues, typically at least 45 amino acid residues, and preferably more than 60 amino acid residues.

The term"fragment" is meant to include polypeptides that are truncations or deletions of SP, pS2 and ITF. Preferably, the fragments have 70% amino acid identity to the corresponding regions of the human polypeptide sequence. More preferably, the fragments are 85% identical, most preferably 95%, or even 99% identical to the human polypeptide sequence to which they correspond. The length of comparison sequences will generally be at least about 10 amino acid residues, usually at least 20 amino acid residues, more usually at least 30 amino acid residues, typically at least 45 amino acid residues, and preferably more than 60 amino acid residues.

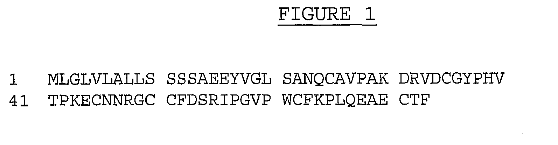

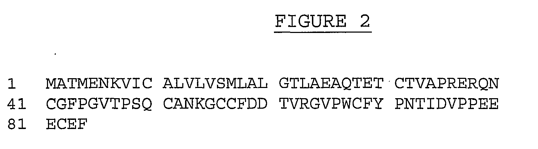

Preferable fragments contain four cysteine residues in any positions which correspond to the cysteines at positions 25,35,45,50,51,62, or 71, of human
ITF (Figure 1), or positions 31,41,51,56,57,68, and 82 of human pS2 (Figure 2). More preferably, fragments contain five cysteine residues at these positions.

Most preferably, six, or even all seven cysteines are present.

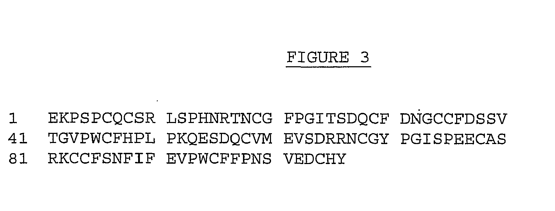

Fragments of SP are meant to include truncations or deletions and preferably have 70% sequence identity to the corresponding human SP polypeptide sequence (Figure 3). More preferably, the fragments are 85% identical, most preferably 95%, or even 99% identical to the human polypeptide sequence. Preferably, active fragments contain at least four cysteine residues which correspond to positions 6,8,19,29,34,35,46,58,68,78,83,84,95, and 104 in the human SP polypeptide. More preferably, fragments contain six cysteines which correspond to these positions. Even more preferable are fragments that contain eight cysteines. Most preferable are fragments that contain cysteines at ten, twelve, or even, all fourteen positions.

It is recognized in the art that one function of the identified cysteine residues is to impart the characteristic three-loop (trefoil) structure to the protein.

Accordingly, preferred fragments of ITF and pS2 have a least one loop structure, more preferably, the fragments have two loop structures, and most preferably, they have three loop structures. It is equally well recognized that the native SP polypeptide has a six loop confirmation. Preferable fragments contain at least two of these loop structures, more preferably, four loop structures are conserved, and most preferably, five, or even all six loop structures are present.

By"co-formulated"is meant any single pharmaceutical composition which contains two or more therapeutic or biologically active agents.

By"pharmaceutical preparation"or"pharmaceutical composition"is meant any composition which contains at least one therapeutically or biologically active agent and is suitable for administration to a patient. For the purposes of this invention, pharmaceutical compositions suitable for delivering a therapeutic to the upper alimentary canal include, but are not limited to solutions and suspensions delivered either as an oral spray or rinse, pastes, gels, chewable tablets, sublingual, gingival, or buccal wafers and films, chewing gum, lozenges, and other compositions designed to be retained in the mouth for an extended period of time. Any of these formulations can be prepared by well known and accepted methods of art. See, for example, Remingtion: The Science and Practice of Pharmacy, 19i edition, (ed. AR Gennaro), Mack Publishing Co., Easton, PA, 1995.

By"microsphere"is meant a bioerodable polymeric pharmaceutical delivery device having a diameter of 5-100llm and a hollow central core suitable for encapsulation of the therapeutic agent. Typically, the therapeutic agent is encapsulated at the time of microsphere formulation.

By"therapeutically effective amount"is meant an amount sufficient to provide medical benefit. When administering trefoil peptides to a human patient according to the methods described herein, a therapeutically effective amount is usually about 0.1-1000 mg of intestinal trefoil peptide per day. Preferably, the patient receives, 10 mg, 100 mg, 250 mg, or 750 mg of intestinal trefoil peptide each day. The total daily does can be divided into multiple individual doses.

By"upper alimentary canal"is meant the portion of the digestive system proximal to the cardiac sphincter (cardioesophageal sphincter) of the stomach.

Specifically, the upper alimentary canal is meant to include the oral cavity and associated structures (e. g., the tongue, gingival and sublingual tissues, and the hard and soft palates) and the esophagus.

By"biologicallyactive,"when referring to an intestinal trefoil peptide, fragment, or homolog is meant any polypeptide that exhibits an activity common to its related, naturally occurring family member, and that the activity is common to the family of naturally occurring intestinal trefoil peptides. An example of a biological activity common to the family of trefoil peptides is the ability to alter gastrointestinal motility in a mammal.

By"isolated DNA"is meant DNA that is free of the genes which, in the naturally-occurring genome of the organism from which the given DNA is derived, flank the DNA. Thus, the term"isolated DNA"encompasses, for example,cDNA, cloned genomic DNA, and synthetic DNA.

By"treating"is meant administering a pharmaceutical composition for prophylactic and/or therapeutic purposes. The active ingredients of the pharmaceutical composition can treat the primary indication (i. e., epithelial lesion) or secondary symptoms (e. g., concomitant infection, pain, or inflammation).

By "analgesic" is meant an agent which relieves pain by elevating the pain threshold without significantly disturbing the consciousness of the patient.

By"antimicrobial agent"is meant any compound that alters the growth of bacteria or fungi cells, or viruses whereby growth is prevented, stabilized, or inhibited, or wherein the microbes are killed. In other words, the antimicrobial agents can be microbiocidal or microbiostatic.

By"thermal burn"is meant injury to or destruction of at least the epithelial cell layer caused by exposure to excessive temperature. Thermal burns of the upper alimentary canal are usually caused by ingestion of overly-heated foods and liquids, or inhalation of super-heated air. Thermal burns are meant to include, but are not limited to, burns classified as first degree, second degree, and third degree burns.

By"chemical burn"is meant injury to or destruction of at least the epithelial cell layer caused by exposure to noxious chemicals. Typically, chemical exposures of the upper alimentary canal are caused by inhalation or ingestion.

By "antineoplastic therapy"is meant any treatment regimen used to treat cancer. Typical antineoplastic therapies include chemotherapy and radiation therapy.

### Brief Description of Drawings

Figure 1 is the amino acid sequence of a human intestinal trefoil factor (ITF; Accession No. BAA95531 ; SEQ ID NO: 1).
Figure 2 is the amino acid sequence of a human pS2 protein (Accession No. NP003216 ; SEQ lD NO: 2).
Figure 3 is the amino acid sequence of human spasmolytic polypeptide (SP; Accession No. 1909187A; SEQ ID NO : 3).
Figure 4 is acDNA sequence encoding a human intestinal trefoil factor (SEQ lD NO: 4).
Figure 5 is a cDNA sequence encoding a human pS2 protein (SEQ ID NO: 5).
Figure 6 is acDNA sequence encoding a human spasmolytic polypeptide (SEQ ID NO: 6).
Figure 7 is the nucleotide sequence of a gene encoding human intestinal trefoil factor (locus 10280533: 52117-55412 ; SEQ lD NO: 7).
Figure 8 is the nucleotide sequence of a gene encoding human pS2 protein (locus 10280533: 16511-21132; SEQ lD NO:8).
Figure 9 is the nucleotide sequence of a gene encoding human spasmolytic polypeptide (locus 10280533: 957-5208; SEQ lD NO: 9).

### Detailed Description

The invention provides methods and compositions useful for the treatment of a wide range of lesions of the upper alimentary canal. The intestinal trefoil peptide therapy of this invention is particularly useful for treating epithelial lesions of the oral and esophageal mucosa, tongue, and gingival tissue.

Mammalian trefoil peptides were discovered in 1982. One of the mammalian trefoil peptides, human intestinal trefoil factor (ITF; TFF3), has been characterized extensively, and is described in U. S. Patent Nos. 6,063,755, and 6,221,840, hereby incorporated by reference. The other two known human intestinal trefoil peptides are spasmolytic polypeptide (SP; TFF2) and pS2(TFF1). Trefoil peptides, described extensively in the literature (e. g., Sands etal., Annu. Rev. Physiol. 58: 253-273 (1996), hereby incorporated by reference), are expressed in the gastrointestinal tract and have a three-loop structure formed by intrachain disulfide bonds between conserved cysteine residues. These peptides protect the intestinal tract from injury and can be used to treat intestinal tract disorders such as peptic ulcers and inflammatory bowel disease. Homologs of these human peptides have been found in a number of non-human animal species.

All members of this protein family, both human and non-human, are referred to herein as trefoil peptides. Human ITF will be referred to most extensively in this application; however, the activity of human ITF is common to each of the mammalian intestinal trefoil peptides.

We have discovered that epithelial lesions of the upper alimentary canal including the oral and esophageal mucosa, tongue, and gingival tissue can be treated by local administration of intestinal trefoil peptides. Thus, trefoil peptide therapy, according to the methods of this invention, can be delivered in any pharmaceutical composition which is useful for delivering therapeutics to the upper alimentary canal.

### Pharmaceutical Preparations

### Oral Sprays, Rinses, and Emulsions

Spray systems are particularly useful for delivering therapeutics to the upper alimentary canal. Suitable spray delivery systems include both pressurized and non-pressurized (pump actuated) delivery devices. The intestinal trefoil peptide-containing solution, delivered as an oral spray, is preferably an aqueous solution; however, organic and inorganic components, emulsifiers, excipients, and agents that enhance the organoleptic properties (i. e., flavoring agents or odorants) may be included. Optionally, the solution may contain a preservative that prevents microbial growth (i. e., methyl paraben). Although water itself may make up the entire carrier, typical liquid spray formulations contain a co-solvent, for example, propylene glycol, corn syrup, glycerin, sorbitol solution and the like, to assist solubilization and incorporation of water-insoluble ingredients. In general, therefore, the compositions of this invention preferably contain from about 1-95% v/v and, most preferably, about 5-50% v/v, of the co-solvent. When prepared as an spray, patients typically self-administer 1-5 times per day. The spray delivery system is normally designed to deliver50-100 1 per actuation, and therapy may require 1-5 actuations per dose. The rheological properties of the spray formulation are optimized to allow shear and atomization for droplet formation.

Additionally, the spray delivery device is designed to create a droplet size which promotes retention on mucosal surfaces of the upper alimentary canal and minimize respiratory exposure.

Compositions suitable for oral sprays can also be formulated as an oral rinse or mouthwash. Administration of trefoil peptides using these formulations is typically done by swishing, gargling, or rinsing the oral cavity with the formulation. Optionally, these formulations can be swallowed, providing trefoil peptide therapy to the esophagus, stomach, and/or intestines. This delivery method is particularly useful for treating patients suffering related disorders of the intestinal epithelium. For example, patients receiving antineoplastic chemotherapy, in addition to oral mucositis, frequently develop more distal lesions of the gastrointestinal tract such as lesions of the gastric and intestinal epithelium. It is well known that intestinal trefoil peptides, particularly ITF, are stable at stomach pH. Thus, swallowing an intestinal trefoil peptide-containing solution designed primarily for treating oral mucositis may also benefit lesions of the lower alimentary canal (i. e., stomach and intestines).

In an alternative formulation, the intestinal trefoil peptides and/or other therapeutics can be encapsulated in bioerodable microspheres rather than being dissolved in the aqueous phase of the formulation. A wide variety of microencapsulation drug delivery systems have been developed and many share similar polymeric compositions as used for bioerodable films (described below).

Polymers commonly used in the formation of microspheres include, for example, polycaprolactone, poly(-caprolactone-Co-DL-lactic acid), poly (DL-lactic acid), poly (DL-lactic acid-Co-glycolic acid) and poly(E-caprolactone-Co-glycolic acid) (see, for example, Pitt etal., J. Pharm. Sci., 68 : 1534,1979).

Microspheres can be made by procedures well known in the art including spray drying, coacervation, and emulsification (see for example Davis et al. Microsphere and Drug Therapy, Elsevier, 1984 ; Benoit et al. Biodegradable Microspheres: Advances in Production Technologies, Chapter 3, Ed. Benita, S, Dekker, New York, 1996; Microencapsulation and Related Drug Processes, Ed. Deasy, Dekker, 1984, New York; U. S. Patent No. 6,365,187). Preferably, the microspheres are bioadhesive or are prepared in formulations containing a bioadhesive excipient.

Other technical features of the intestinal trefoil peptide-containing solutions are easily modified to suit the specific pharmaceutical formulation and the clinical indication being treated. For example, the pH and osmolality of the formulation may be adjusted to confer trefoil peptide stability, while minimizing oral irritancy and sensitivity.

### Ointments, Pastes, and Gels

Lesions of the oral and esophageal epithelium caused by trauma are amenable to trefoil peptide therapy delivered as an ointment, paste, or gel. The viscous nature of these types of preparations allows for direct application into the wound site. Optionally, the wound site can be covered with a dressing to retain the trefoil peptide-containing composition, protect the lesion from trauma,and/or absorb exudate. As discussed further below, these preparations are particularly useful to restore epithelial integrity following traumatic surgical procedures such as, for example, tooth extraction, tissue biopsy, or a tumor resection. Such viscous formulations may also have a local barrier effect thereby reducing irritation and pain.

### Mucoadhesives

A mucoadhesive excipient can be added to any of the previously described pharmaceutical compositions. The mucoadhesive formulations coat the upper alimentary canal providing protection, inhibiting irritation, and accelerating healing of inflamed or damaged tissue. Mucoadhesive formulations also promote prolonged contact of the intestinal trefoil peptide with the mucosal epithelium.

Mucoadhesive formulations suitable for use in pharmaceutical preparations delivered by mouth are well known in the art (e. g., U. S. Patent No. 5,458,879).

Particularly useful mucoadhesives are hydrogels composed of about 0.05-20% of a water-soluble polymer such as, for example, poly (ethylene oxide), poly (ethylene glycol), poly (vinyl alcohol), poly (vinyl pyrrolidine), poly (acrylic acid), poly (hydroxy ethyl methacrylate), hydroxyethyl ethyl cellulose, hydroxy ethyl cellulose, chitosan, and mixtures thereof. These polymeric formulations can also contain a dispersant such as sodium carboxymethyl cellulose (0.5-5.0%).

Other preferred mucoadhesive excipients for liquid compositions are ones that allow the composition to be administered asa flowable liquid but will cause the composition to gel in the upper alimentary canal, thereby providing a bioadhesive effect which acts to hold the therapeutic agents at the lesion site for an extended period of time. The anionic polysaccharides pectin and gellan are examples of materials which when formulated into a suitable composition will gel in the upper alimentary canal, owing to the presence of cations in the mucosal and salivary fluids. The liquid compositions containing pectin or gellan will typically consist of 0.01-20% w/v of the pectin or gellan in water or an aqueous buffer system.

Other useful compositions which promote mucoadhesion and prolonged therapeutic retention in the upper alimentary canal are colloidal dispersions containing 2-50% colloidal particles such as silica or titanium dioxide. Such formulations form as a flowable liquid with low viscosity suitable as a mouthwash or for generating a fine mist. However, the particles interact with glycoprotein, especially mucin, transforming the liquid into a viscous gel, providing effective mucoadhesion (e. g., U. S. Patent Nos. 5,993,846 and 6,319,513).

### Bioerodable Film Delivery Devices

The most simple bioerodable devices contain the therapeutic agent (s) incorporated into a solid, usually lipid-containing, film or tablet. The device is formulated to remain solid at room temperature, but melt at body temperature, releasing the incorporated therapeutics. Suitable formulations of this type include, for example, cocoa butter.

Polymeric film devices provide several advantages for therapeutic delivery to the oral cavity. Unlike rinses, pastes, gels, and other flowable compositions, a film device can reside for prolonged periods of time (i. e., hours to days) in the oral cavity and provide sustained release throughout its residency. Typically, the film is partially or completely bioerodable and contains a mucoadhesive layer to fasten the film to the oral mucosa. Film devices, in addition to its use for delivering therapeutics, can also provide protection against mechanical injury or microbial infection of a lesion site. This physical barrier function is particularly advantageous when treating conditions such as mucositis or aphthous stomatitis.

Additionally, as discussed further below, a film device can be used to release trefoil peptide therapy directly onto the underlying mucosa, into the lumen of the oral cavity, or a combination of both.

Film devices consist of at least two layers; a mucoadhesive layer suitable for attaching the film to the oral mucosa and a bulk layer which contains the active therapeutic (s). Many suitable mucoadhesives are known in the art and are discussed above. Optionally, one or more therapeutics can also be provided in the adhesive layer.

The bulk layer of the composite delivery device may be made of one or more bioerodable polymeric materials. Suitable polymers include, for example, starch, gelatin, polyethylene glycol, polypropylene glycol, polyethylene oxide, copolymers of ethylene oxide and propylene oxide, copolymers of polyethylene glycol and polypropylene glycol, polytetramethylene glycol, polyether urethane, hydroxyethyl cellulose, ethyl cellulose, hydroxypropyl cellulose, hydroxypropylmethyl cellulose, alginate, collagen, polylactide, poly(lactide-co- glycolide) (PLGA), calciumpolycarbophil, polyethymethacrylate, cellulose acetate, propylene glycol, polyacrylic acid, crosslinked polyacrylic acid, hydroxyethylmethacrylate/methyl methacrylate copolymer, silicon/ethyl cellulose/polyethylene glycol, urethane polyacrylate, polystyrene, polysulfone, polycarbonate, polyorthoesters, polyanhydrides, poly (amino acids), partially and completely hydrolyzed alkylene-vinyl acetate copolymers, polyvinyl chloride, polymers of polyvinyl acetate, polyvinyl alkyl ethers, styrene acrylonitrile copolymers, poly (ethylene terphthalate), polyalkylenes, poly (vinyl imidazole), polyesters and combinations of two or more of these polymers.

A particularly useful bulk layer polymer consists of PLGA and ethyl cellulose. PLGA is bioerodable and can be formulated to degrade over a wide range of conditions and rates. Ethyl cellulose is a water-insoluble polymer that can act as a plasticizer for the PLGA when a film is formed, but will be eroded in a bodily fluid. Due to its water-insolubility, it also has an effect on the degree and rate of swelling of the resultant film.

An optional third layer which is impermeable to the trefoil peptide can also be added to the wafer. Preferably, this barrier layer is also bioerodable. Suitable barrier layer polymers include ethyl cellulose, poly (acrylic acid), or otherpolyelectrolytes. In one configuration, the barrier layer is placed on the opposite side of the bulk layer relative to the adhesive layer, thereby directing the released therapeutic agent onto the contacted epithelium rather than being diluted in the lumenal fluid. This configuration is particularly useful for treating discrete lesions (i. e., mucositis or aphthous stomatitis) of the tongue, sublingual tissue, or buccal mucosa. In an alternative configuration of the film device, the barrier layer is placed between the bulk layer and the adhesive layer. This configuration directs therapeutic release into the lumen of the oral cavity and is useful for treating more diffuse lesions of the tongue, oral cavity, and esophagus. The configuration is also useful for delivering therapeutics which are cytotoxic when administered at high concentrations because it has the effect of shielding the underlying tissue from direct contact with the therapeutic-containing film.

### Chewable Tablets, Lozenges, and Confectionaries

Preparing a trefoil peptide-containing composition as a chewable tablet, lozenge, or a confectionary such as chewing gum provides several advantages to traditional drug delivery vehicles. First, prolonged contact and sustained release at the target site (mouth and esophagus) is achieved. Second, such formulations often results in higher patient compliance, especially when administering trefoil peptides to children.

Formulations for chewable tablets are well known and typically contain a base of sugar, starch, or lipid and a flavoring agent. An exemplary formulation for a chewable tablet is provided below.

Chewable ITF Tablet Formulation (per tablet)
Intestinal trefoil factor-300 mg
Mannitol-675 mg
Microcrystalline cellulose-75mg
Corn starch-30 mg
Calcium sterate-22 mg
Flavoring Agent (i. e., sodium saccharin or peppermint oil)
The incorporation of therapeutics into chewing gum and other confectionary style formulations is known in the art (e. g., U. S. Patent No.
5, 858, 391).
Therapeutics Agents
Trefoil Peptides
In preferred embodiments, the trefoil peptide is a human trefoil peptide.

More preferably, it is human intestinal trefoil factor (ITF), spasmolytic polypeptide (SP), or pS2. Most preferably, the trefoil peptide is human ITF.

The trefoil peptides are present in the compositions of the invention at a concentration of between 0.1-1000 mg/ml, depending on the nature and condition of the lesion being treated, the anticipated frequency and duration of therapy, and the type of pharmaceutical composition used to deliver the trefoil peptide.

Typically, therapy is designed to deliver 0.1-500 mg of trefoil peptide per day to the patient.

### Anti-Inflammatory Agents

Any suitable anti-inflammatory agent can be formulated in the compositions of the invention, at concentrations known for these agents. Many of the most useful anti-inflammatory agents also have analgesicand/or antipyretic properties. Anti-inflammatory agents suitable forco-formulation with a trefoil peptide include, for example, acetaminophen, aspirin (acetylsalicylic acid), ibuprofen, phenylbutazone, indomethacin, sulindac, diclofenac, and naproxen.

### Antimicrobial Agents

Any of the many known microbial agents can be used in the compositions of the invention at concentrations generally used for these agents. Antimicrobial agents include antibacterials, antifungals, antivirals, and other topical antiseptics.

Examples of antibacterial agents (antibiotics) include the penicillins (e. g., penicillin G, ampicillin, methicillin, oxacillin, and amoxicillin), the cephalosporins (e. g., cefadroxil, ceforanid,cefotaxime, and ceftriaxone), the tetracyclines (e. g., doxycycline, minocycline, and tetracycline), the aminoglycosides (e. g., amikacin, gentamycin, kanamycin, neomycin, streptomycin, and tobramycin), the macrolides (e. g., azithromycin, clarithromycin, and erythromycin), the fluoroquinolones (e. g., ciprofloxacin,lomefloxacin, and norfloxacin), and other antibiotics including chloramphenicol, clindamycin, cycloserine, isoniazid, rifampin, and vancomycin.

Antiviral agents are substances capable of destroying or suppressing the replication of viruses. Examples of anti-viral agents include1,-D-ribofuranosyl- 1, 2,4-triazole-3 carboxamide,9- > 2-hydroxy-ethoxy methylguanine, adamantanamine, 5-iodo-2'-deoxyuridine, trifluorothymidine, interferon, adenine arabinoside, protease inhibitors, thymadine kinase inhibitors, sugar or glycoprotein synthesis inhibitors, structural protein synthesis inhibitors, attachment and adsorption inhibitors, and nucleoside analogues such as acyclovir, penciclovir, valacyclovir, and ganciclovir.

Antifungal agents include both fungicidal and fungistatic agents such as, for example, amphotericin B, butylparaben, clindamycin, econaxole, fluconazole, flucytosine, griseofulvin, nystatin, and ketoconazole.

Topical antiseptics include agents such as, for example, povidone-iodine and benzalkonium chloride.

### Analgesic and Anesthetics

Any of the commonly used topical analgesics can be used in the compositions of the invention. The analgesic is present in an amount such that there is provided to the oral lesion a topical concentration of between one-half and five percent concentration for lidocaine (5-50 mg/ml in 20-40 ml per dose of liquid). Examples of other useful anesthetics include procaine, lidocaine, tetracaine,dibucaine, benzocaine, p-buthylaminobenzoic acid 2- (diethylamino) ethyl ester HC1, mepivacaine, piperocaine, and dyclonine.

Other analgesics include opioids such as, for example, morphine, codeine, hydrocodone, and oxycodone. Any of these analgesics may also beco-formulated with other compounds having analgesic or anti-inflammatory properties, such as acetaminophen, aspirin, and ibuprofen.

### Steroids

Steroids are commonly used to treat lesions of the upper alimentary canal.

For example, oral aphthous stomatitis is typically treated using a paste preparation of triamcinolone (0.1%), hydrocortisone, fluticasone,or beclomethasone.

### Conditions of the Upper Alimentary Canal Treated Using Trefoil Peptides Mucositis

Mucositis is a common condition of the oral cavity which is characterized by inflammation of the mucous membranes. The condition is frequently caused by antineoplastic therapy, including chemotherapy and local radiation therapy.

Symptoms of mucositis include ulcerations, redness, and swelling, and is associated with epithelial cell injury and death. Patients suffering from severe mucositis are susceptible to dehydration and malnutrition because mucositis pain limits dietary intake. In severe cases, mucositis can be so debilitating that patients may require prolonged hospitalization, parenteral nutrition, and narcotic pain medication. Additionally, destruction of the mucosal epithelium increases a patient's susceptibility to local and systemic infection. Disruption of the barrier function permits entry of microorganisms and microbial products normally retained in the gut lumen. Thus, pharmaceutical preparations which reduce the adverse effects associated with chemotherapy will improve the patient's quality of life, compliance with self-medication, and may permit administration of higher chemotherapeutic doses. Typically, mucositis is treated using a trefoil peptidecontaining rinse or oral spray which the patient self-administers 1-5 times per day. The aqueous solution preferably contains a mucoadhesive and an antiinflammatory agent. Other therapeutics, such as an topical analgesic agent (e. g., lidocaine) may also be present. Alternatively, if the lesions are few in number and spatially localized, an intestinal trefoil peptide-containing film device an be placed directly over the lesions.

### Tooth Extraction

Intestinal trefoil peptide-containing compositions of the invention are used to lessen complications and speed healing of the wound created by the extraction of a tooth. An oral rinse, paste, ointment, or gel, as described above, is applied to the site of extraction immediately following the procedure and then 1-4 times per day, as needed, until epithelial regrowth is complete. Preferably, a topical analgesic is included in the formulation to relieve the temporary discomfort cause by the trauma of extraction. As a prophylactic measure, antibiotic agents may also be included in the formulation.

### Gingivitis

Gingivitis is most commonly a chronic disease requiring ongoing treatment, in some cases for months or even years. The trefoil peptide-containing compositions of the invention can be employed to treat gingivitis, alone or in conjunction with other treatments, particularly with an anti-microbial agent, and most commonly with an antibacterial agent. An oral intestinal. trefoil peptidecontaining rinse is swished in the patient's mouth at least once every 2-3 days, but as often as thrice daily, over a 3-4 week period, and the regimen is repeated as needed. Alternatively, the trefoil peptide is formulated into a gel or toothpaste.

In severe cases, a viscous gel or ointment having a high intestinal trefoil peptide concentration is applied directly to the wound via a pledget with a stick applicator.

Intestinal trefoil peptide-containing compositions can also be delivered in biodegradable drug delivery systems capable of formation of films applied below the gum line (described in U. S. Patent Nos. 5,945,115 and 5,990,194. A biodegradable polymer, admixed with the intestinal trefoil peptide, is provided where the polymer can be injected in as a free-flowing solution below the gum line using a syringe. The polymer solution then, insitu, forms a solid biodegradable implant.

### Aphthous Stomatitis

At the first indication of an outbreak of aphthous stomatitis (generally, the first twinge of pain), the patient swishes the mouth with an intestinal trefoil peptide-containing rinse, 1-4 times per day until the ulcer heals (generally 5-10 days). An intestinal trefoil peptide-containing gel can also be applied to the ulcer, in the same manner that steroid-containing gels are currently used. In addition, a gel can contain both an intestinal trefoil protein and a steroid known to be effective for aphthous stomatitis treatment. A direct application of more concentrated material can be directly applied to the wound via a pledget with a stick applicator. Alternatively, the lesion can be treated directly by applying a bioerodable film device containing both a trefoil peptide and a steroid (i. e., triamcinolone) directly to the lesion. Any formulation useful for treating aphthous stomatitis can also, optionally, contain a local anesthetic (i. e., lidocaine or benzocaine).

### Behcet's Disease

Behcet's Disease is a rare, multi-system rheumatic disorder characterized by systemic vasculitis. One of the most frequent symptoms of Behcet's Disease is recurrent oral ulcerations which resemble aphthous lesions. Currently, treatment for Behcet's Disease is palliative, not curative. Thus, the intestinal trefoil peptides can be used to treat lesions of the upper alimentary canal in conjunction with currently available Behcet's Disease therapies including, for example, interferon alpha 2A and 2B, levamisole,cyclosporine, cyclophosphamide, and colchicine.

### Oral Biopsy and Oral Surgery

In cases in which an oral neoplasm is suspected or known to be malignant, a biopsy or a curative resection is performed using a needle or a scalpel, resulting in an open wound. The surgical area, susceptible to infection and inflammation, is treated by rinsing with a trefoil peptide-containing solution1-4 times per day.

Preferably, an analgesic, an anti-inflammatory, and an antibiotic are included in the formulation. Alternatively, a more concentrated gel, paste, or ointment may be directly applied to the lesion site. For post-operative treatment following resection of a malignancy, a topically active chemotherapeutic can be including in the trefoil peptide-containing composition.

### Thermal and Chemical Burns

Trauma to the upper alimentary canal is frequently caused by exposure to excessive heat or noxious chemicals. Thermal burns to the upper alimentary canal are frequently mild in nature (i. e., first or second degree burns), resulting from the ingestion of overheated food or drink. More severe thermal bums of the oral mucosa and upper esophagus can be caused by inhalation of super heated air and are frequently observed infirefighters or victims of house or forest fires.

Chemical exposure can also damage the mucosa of the upper alimentary canal. Mild mucosal irritations and burns are often caused by ingestion of acidic food (i. e., fruits). More severe chemical burns are usually associated with accidental industrial or occupational exposures.

The intestinal trefoil peptide-containing pharmaceutical formulations described herein are useful for treating thermal and chemical burns of the upper alimentary canal. Preferably, viscous liquid or gel formulation containing a mucoadhesive is used to prolong mucosal exposure to the trefoil peptide.

Alternatively, a sustained release formulation, such as a bioerodable film, is used.

Topical analgesics and antimicrobial agents are the most preferred secondary therapeutics to be co-administered.

### Production of Intestinal Trefoil Peptides

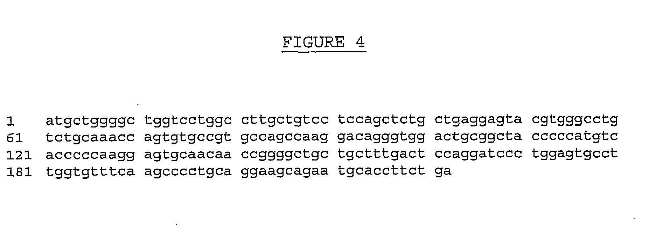

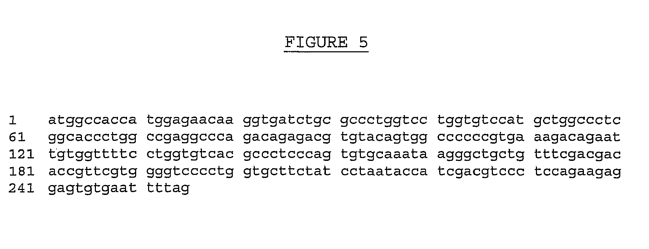

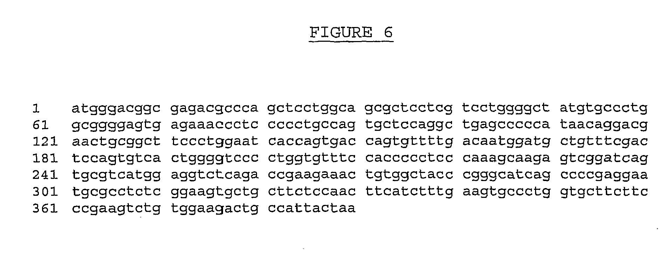

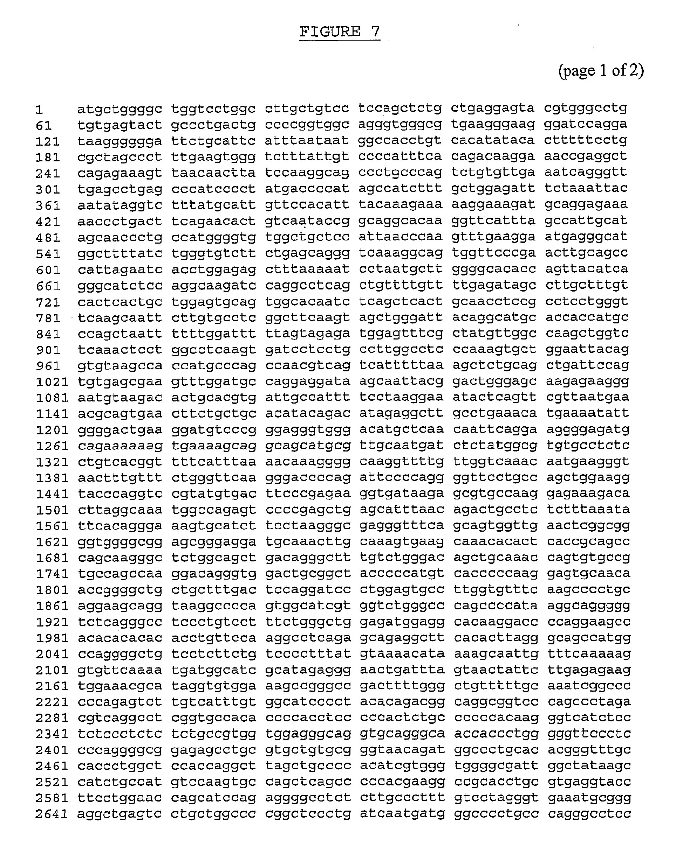

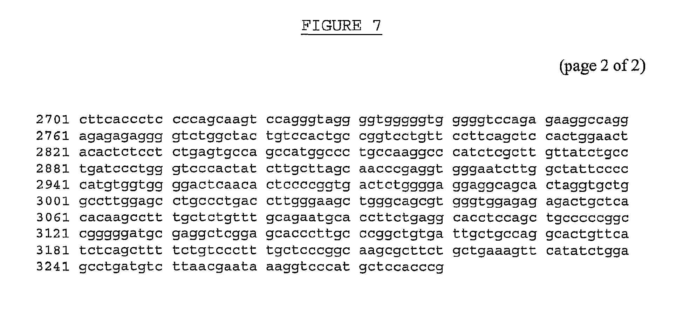

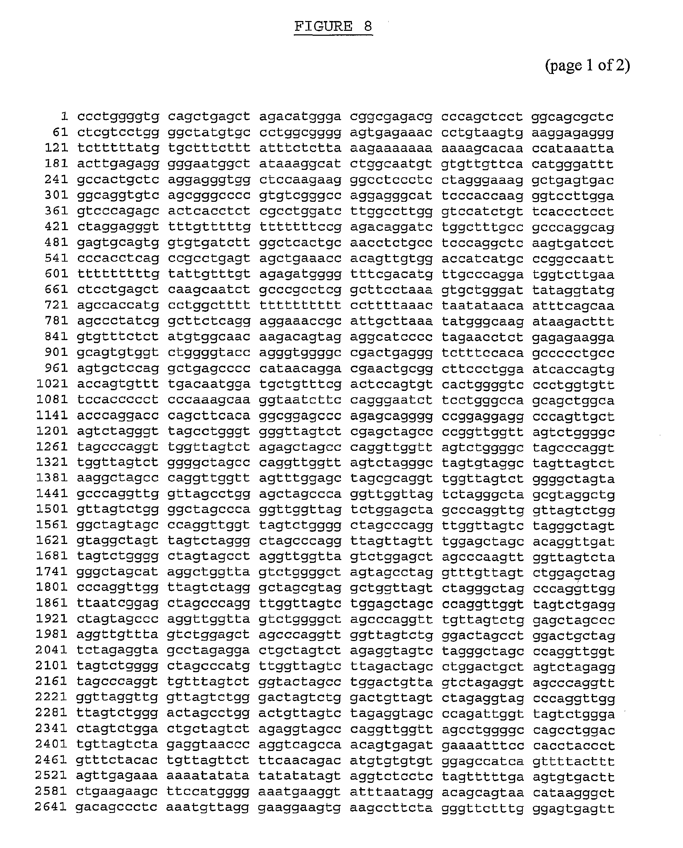

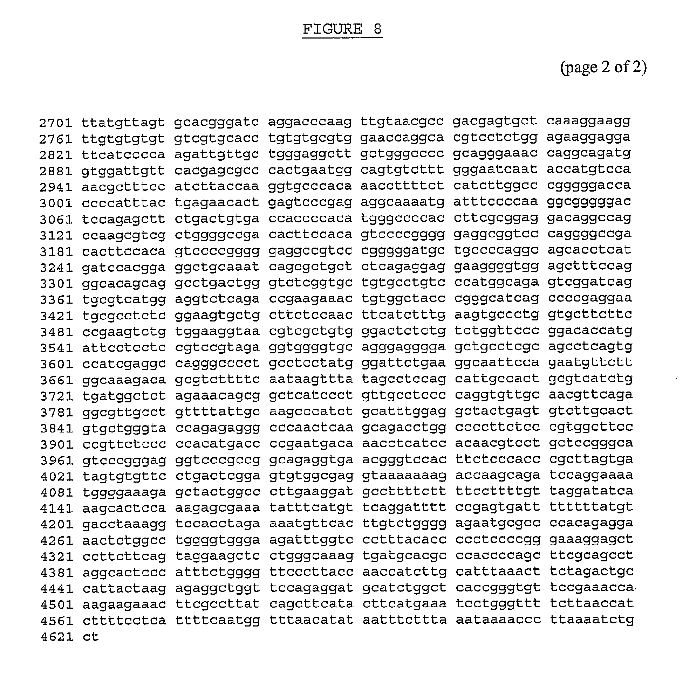

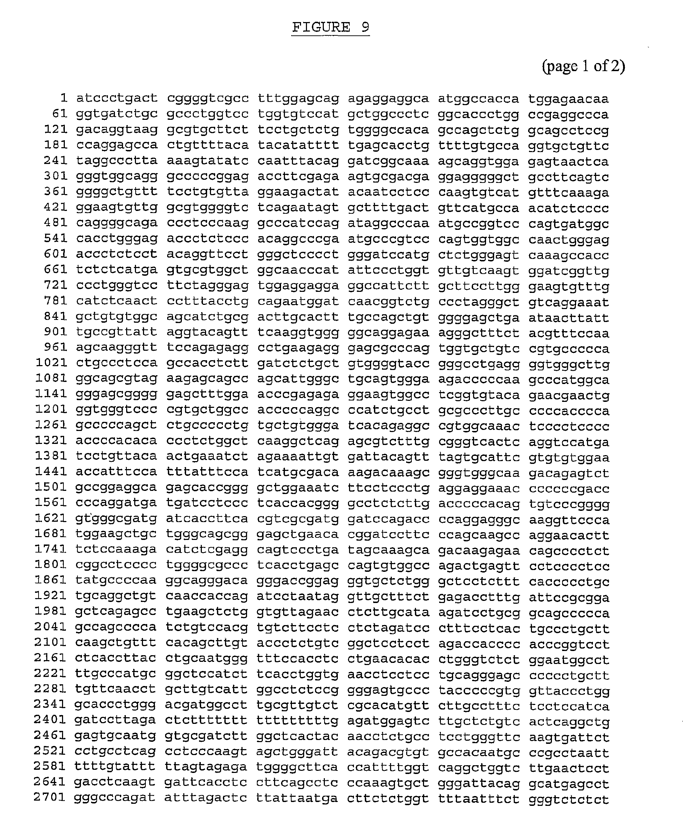

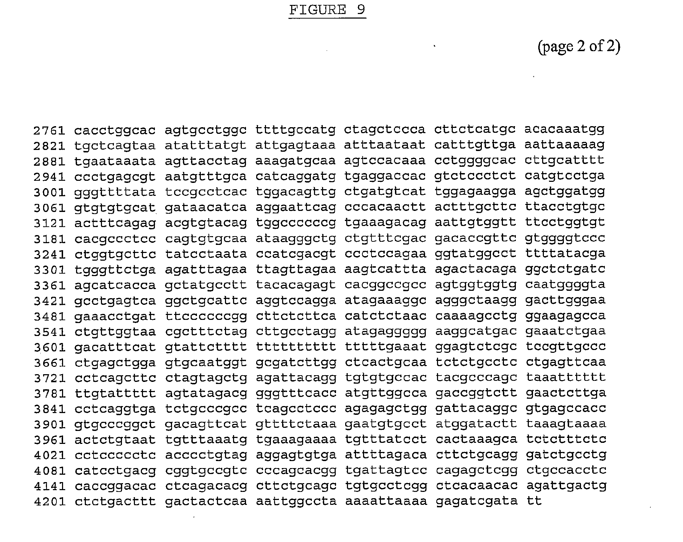

Intestinal trefoil peptides can be produced by any method known in the art for expression of recombinant proteins. Nucleic acids that encode trefoil peptides (e. g., human intestinal trefoil factor (Figure 4 and 7), human pS2 (Figure 5 and 8), and human spasmolytic polypeptide (Figure 6 and 9) or fragments thereof may be introduced into various cell types or cell-free systems for expression thereby allowing large-scale production, purification, and patient therapy.

Eukaryotic and prokaryotic trefoil peptide expression systems may be generated in which an intestinal trefoil peptide gene sequence is introduced into a plasmid or other vector, which is then used to transform living cells. Constructs in which the intestinal trefoil peptidecDNA contains the entire open reading frame inserted in the correct orientation into an expression plasmid may be used for protein expression.Prokaryotic and eukaryotic expression systems allow for the expression and recovery of intestinal trefoil peptide fusion proteins in which the trefoil peptide is covalently linked to a tag molecule which facilitates identification and/or purification. An enzymatic or chemical cleavage site can be engineered between the trefoil peptide and the tag molecule so that the tag can be removed following purification.

Typical expression vectors contain promoters that direct the synthesis of large amountsof mRNA corresponding to the inserted intestinal trefoil peptide nucleic acid in the plasmid-bearing cells. They may also include a eukaryotic or prokaryotic origin of replication sequence allowing for their autonomous replication within the host organism, sequences that encode genetic traits that allow vector-containing cells to be selected for in the presence of otherwise toxic drugs, and sequences that increase the efficiency with which the synthesizedmRNA is translated. Stable long-term vectors may be maintained as freely replicating entities by using regulatory elements of, for example, viruses (e. g., the
OriP sequences from the Epstein Barr Virus genome). Cell lines may also be produced that have integrated the vector into the genomic DNA, and in this manner the gene product is produced on a continuous basis.

Expression of foreign sequences in bacteria, such asEscherichia coli, requires the insertion of an intestinal trefoil peptide nucleic acid sequence into a bacterial expression vector. Such plasmid vectors contain several elements required for the propagation of the plasmid in bacteria, and for expression of the
DNA inserted into the plasmid. Propagation of only plasmid-bearing bacteria is achieved by introducing, into the plasmid, selectable marker-encoding sequences that allow plasmid-bearing bacteria to grow in the presence of otherwise toxic drugs. The plasmid also contains a transcriptional promoter capable of producing large amountsof mRNA from the cloned gene. Such promoters may be (but are not necessarily) inducible promoters that initiate transcription upon induction.

The plasmid also preferably contains a polylinker to simplify insertion of the gene in the correct orientation within the vector. Mammalian cells can also be used to express a trefoil peptide. Stable or transient cell line clones can be made using intestinal trefoil peptide expression vectors to produce the trefoil peptides in a soluble (truncated and tagged) form. Appropriate cell lines include, for example,
COS, HEK293T, CHO, or NIH cell lines.

Once the appropriate expression vectors are constructed, they are introduced into an appropriate host cell by transformation techniques, such as, but not limited to, calcium phosphate transfection, DEAE-dextran transfection, electroporation, microinjection, protoplast fusion, or liposome-mediated transfection. The host cells that are transfected with the vectors of this invention may include (but are not limited to) E. coli or other bacteria, yeast, fungi, insect cells (using, for example, baculoviral vectors for expression in SF9 insect cells), or cells derived from mice, humans, or other animals. In vitro expression of trefoil peptides, fusions, or polypeptide fragments encoded by cloned DNA may also be used. Those skilled in the art of molecular biology will understand that a wide variety of expression systems and purification systems may be used to produce recombinant trefoil peptides and fragments thereof. Some of these systems are described, for example, in Ausubel et al. (Current Protocols in Molecular Biology, John Wiley & Sons, New York, NY 2000, hereby incorporated by reference).

Transgenic plants, plant cells and algae are also particularly useful for generating recombinant intestinal trefoil peptides for use in the methods and compositions of the invention. For example, transgenic tobacco plants or cultured transgenic tobacco plant cells expressing an intestinal trefoil peptide can be created using techniques known in the art (see, for example, U. S. Patent Nos.
5,202,422 and 6,140,075). Transgenic algae expression systems can also be used to produce recombinant intestinal trefoil peptides (see, for example, Chen et al., Curr. Genet. 39: 365-370,2001).

Once a recombinant protein is expressed, it can be isolated from cell lysates using protein purification techniques such as affinity chromatography.

Once isolated, the recombinant protein can, if desired, be purified further by e. g., high performance liquid chromatography (HPLC; e. g., see Fisher, Laboratory Techniques In Biochemistry And Molecular Biology, Work and Burdon, Eds., Elsevier,1980).

Polypeptides of the invention, particularly short intestinal trefoil peptide fragments can also be produced by chemical synthesis using, for example,
Merrifield solid phase synthesis, solution phase synthesis, or a combination of both (see, for example, the methods described in Solid Phase Peptide Synthesis, 2nd ed., 1984, The Pierce Chemical Co.,Rockford, IL). Optionally, peptide fragments are then be condensed by standard peptide assembly chemistry.

### Example 1 : Mucositis Treatnzentror Patients Receiving Antineoplastic

### Therapy

Trefoil peptide therapy is initiated prior to antineoplastic therapy (i. e., chemotherapy or radiation therapy), as a prophylactic to delay or prevent the onsetof mucositis. Preferably, the patient begins intestinal trefoil peptide therapy three days prior to the first dose of antineoplastic therapy. During the prophylactic stage, the patient rinses the oral cavity with an intestinal trefoil peptide-containing solution. Alternatively, for convenience, the trefoil peptide is provided as a concentrated oral spray. Preferably, the patient swallows the solution, providing protection for the epithelial cells of the esophagus and lower gastrointestinal tract.

Rinsing with and swallowing the intestinal trefoil peptide-containing solution continues at least twice daily until oral or esophageal mucositis is detected.

In patients with existing mucositis, epithelial healing is promoted using intestinal trefoil peptide therapy as described above. Palliative therapy is provided using benzocaine (a local anesthetic), and nystatin (an antifungal). The intestinal trefoil peptide can beco-formulated with the benzocaine and nystatin.

For example, the patient swishes an oral rinse solution (mouthwash), containing all therapeutic agents, 1-5 times each day. Alternatively, the trefoil peptide can be provided in a concentrated oral spray, with or without benzocaine and the nystatin is administered in an oral rinse.

The oral rinse solutions can either be swallowed or spit out. If swallowed, an antacid may also be included in the formulation. Other useful therapeutics which provide palliative therapy include antiinflammatories (e.g., ibuprofen) and other anti-microbial agents. Exemplary oral rinses useful for treating chemotherapy-induced mucositis are provided below, but are not intended to be limiting. A skilled physician or pharmacist will immediately recognize appropriate substitutions, additions, and deletions that can be made to these formulations.

Rinse#I : Mix equal partsof : (i) diphenhydramine elixir(Benadryl (g)) (ii) kaolin-pectin suspension(KaopectateM)) (iii) viscous lidocaine HCl (2%) (iv) nystatin (oral suspension; 100,000 iu/ml) (v) ITF (2.5 mg/ml) preferably swallowed after swishing Rinse#2 : Mix equal partsof : (i) diphenhydramine elixir(Benadryl (g) (ii)Maalox (MgOH & A1OH ; 40 mg/ml) (iii) viscous lidocaineHC1 (2%) (iv) ITF (2.5 mg/ml) preferably swallowed after swishingOtlier Embodiments
All publications and patent applications cited in this specification are herein incorporated by reference as if each individual publication or patent application were specifically and individually indicated to be incorporated by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. The use of an intestinal trefoil peptide in the manufacture of a medicament for the treatment of oral mucositis in a mammal.

2. The use of an intestinal trefoil peptide in the manufacture of a medicament for the treatment of aphthous stomatitis in a mammal.

3. The use of an intestinal trefoil peptide in the manufacture of a medicament for the treatment of a lesion of the upper alimentary canal of a mammal, said lesion caused by antineoplastic therapy.

4. The use of an intestinal trefoil peptide in the manufacture of a medicament for the treatment of gingivitis in a mammal.

5. The use of an intestinal trefoil peptide in the manufacture of a medicament for the treatment of a thermal or chemical bum in the upper alimentary canal of a mammal.

6. The use of an intestinal trefoil peptide in the manufacture of a medicament for the treatment of Behcet's disease in a mammal.

7. The use of any of claims 1-6, wherein said intestinal trefoil peptide is:
- spasmolytic polypeptide, pS2, intestinal trefoil factor or biologically active fragment thereof,
- intestinal trefoil factor, or
- a biologically active fragment of intestinal trefoil factor, wherein said fragment comprises a trefoil structure.

8. The use of any of the preceding claims, wherein said mammal is a human.

9. The use of any of the preceding claims, wherein said medicament further comprises a second therapeutic agent, said second therapeutic agent being preferably an anti-inflammatory agent.

10. The use of claim 9, wherein said second therapeutic agent is an antibacterial agent, chosen among a penicillin, a cephalosporin, a tetracycline, an aminoglycoside or povidone-iodine.

11. The use of claim 9, wherein said second therapeutic agent is an anti-fungal agent, preferably nystatin or Amphotericin B.

12. The use of claim 9, wherein said second therapeutic agent is an anti-viral agent, preferably acyclovir.

13. The use of claim 9, wherein said second therapeutic agent is an analgesic, preferably lidocaine or benzocaine.

14. The use of claim 9, wherein said second therapeutic agent is a steroid, preferably triamcinolone or hydrocortisone.

15. A composition suitable for therapeutic delivery to the upper alimentary canal of a mammal, wherein said composition is an oral spray, oral rinse, bioerodable film, or chewing gum comprising an intestinal trefoil peptide.

16. The composition of claim 15, wherein said intestinal trefoil peptide is spasmolytic polypeptide, pS2, intestinal trefoil factor or a biologically active fragment of intestinal trefoil factor, wherein said fragment comprises a trefoil structure.

17. The composition of any of claims 15 and 16, wherein said composition further comprises a mucoadhesive agent or a second therapeutic agent, said second therapeutic agent being preferably an anti-inflammatory agent or an antibacterial agent.

18. The composition of claim 17, wherein said antibacterial agent is a penicillin, a cephalosporin, a tetracycline, an aminoglycoside or povidone-iodine.

19. The composition of claim 17, wherein said second therapeutic agent is an anti-fungal agent, preferably nystatin or Amphotericin B.

20. The composition of claim 17, wherein said second therapeutic agent is an anti-viral agent, preferably acyclovir.

21. The composition of claim 17, wherein said second therapeutic agent is an analgesic, preferably lidocaine or benzocaine.

22. The composition of claim 17, wherein said second therapeutic agent is a steroid, preferably triamcinolone or hydrocortisone.
